Europäisches Patentamt

**European Patent Office**

Office européen des brevets

(19)

(11) Publication number: **0 166 010**
A1

(12)

# EUROPEAN PATENT APPLICATION

(21) Application number: **84107244.0**

(22) Date of filing: **23.06.84**

(51) Int. Cl.⁴: **A 61 B 5/14**

(43) Date of publication of application: **02.01.86**
**Bulletin 86/1**

(84) Designated Contracting States: **AT BE CH DE FR GB LI LU NL SE**

(71) Applicant: **Merighi, Giuseppe, Via Laghi 29/5, Faenza (Ravenna) (IT)**

(72) Inventor: **Merighi, Giuseppe, Via Laghi 29/5, Faenza (Ravenna) (IT)**

(74) Representative: **Sassatelli, Franco, INIP via Ruggi 5, I-40137 Bologna (IT)**

(54) Device for the instantaneous sampling of the blood polluted by venomous reptiles bite.

(57) The instrument is prearranged for sampling by pushing the piston (1) into the cylinder (7).

This action compresses the spring (5) fitted between the collars (4, 6) of the piston rod (2) and cylinder (7).

The aspiration mouth (10) is then fitted over the sore part, which has been previously lanced, and the piston rod (2) is released allowing the piston (1) to be returned by the effect of the spring (5).

The reduction in air pressure in the suction chamber (9) causes the instantaneous drawing of blood from the sore part.

EP 0 166 010 A1

0166010

- 1 -

Device for the instantaneous sampling of the blood polluted by venomous reptiles bite.

The invention refers to an instrument with manual handling permitting the sampling of the polluted blood, with instantaneous intervention, from the wounded part in a quantity which can be regulated. The invented device allows special employs in the emergency intervention in the case of venemous reptile bites, but it can be employed even against mosquitos, wasps, weaver fishes, forest flies, mites, and against insect bites or other injectors of polluting substances on epidermic parts.

The presence of vipers in the Mediterranean regions, particularly in impervious and meadow lands, represents a constant uneasy care and potential danger that can be foreseen beforehand for the persons crossing through, in particular hunters, countrymen, excursionists, gatherers of wood and meadow products. The present therapeutics foresee two subsequent interventions: an emergency one, which can be performed even by the same person who has been injured and tends to draw out the largest possible quantity of polluted blood, and the other one carried out by the physician with clinical means.

For the emergency intervention, if a limb has been hit, at first the blood circulation in the bite zone must be stopped by binding the upper part through a band. The largest possible quantity of polluted blood should then be drawn out by setting a sucker previously compressed bet-

0166010

ween the fingers on the blessed part which can be localized by means of the two bores caused by the teeth; by the teeth; by taking its normal arrangement again through expansion, the sucker draws out a certain quantity of blood. It is advisable to point out that the emergency intervention is possible because this type of venom shows a higher density than blood: consequently, by blocking the latter circulation, it shows a slow absorption and remains for a certain time localized near this part. This enables an efficient intervention in the time immediately following the bite. The present system of blood extraction by means of single suckers that can be previously arranged by means of manual compression, however, permits but to draw out an extremely small part of the polluted blood. In order to try and increase the extraction capacity, the present devices foresee two suckers to be applied successively the one after the other. However, the failing continuity between the two interventions, as well as the modest drawing possibilities of these means, as already mentioned, allow to draw but a part of the venom. Even by using the viper serum, the admission of the hit person to a clinic is necessary. There is to add that fitting the sucker on the part requires a manual force for contracting it, which means some difficulty.

The invented device enables even the blessed person, after having blocked the blood circulation, when a limb has been hit, by means of a band and by lancing the interested part, to easily draw out the quantity of blood which is deemed necessary and, therefore, permitting to draw out nearly all stationing infected blood: this substantially permits to obviate the consequences of the bite. The proceeding can be generically employed in the immediate interventions in the case of animal and insect bited and stings in general.

The instrument employs a cylindrical part in which a seal piston is introduced in initial position on the upper side, to be manually acted on the thrust rod supporting it. The retaining condition is ensured by a helical spring fitted on the said rod between the collors of the thrust base and of the cylinder. When a limb has been injured, after

0166010

having blocked the blood circulation in the part by binding it with a
-band and after having lanced the interested zone, the operator brings
the retention spring under charge by introducing the pressed piston
through the thumb action on the thrust base, while keeping the cylinder on its collar between two fingers. He then fits the cylinder sucking opening by pressure on the hit part and allows the piston to return in its position through its retention spring. The reduction of the
air pressure in the sucking chamber of the cylinder causes the instantaneous coming out of some blood quantity according to the cylinder dia
meter  and to the piston return course. This permits to determine the
quantity of blood to drw out by foreseeing a relevant graduation for
this purpose. In order to allow the blood drawing out from curved epidermic parts, such as they can be considered on the limbs, a cylindrical spout is foreseen fitted with a bent sucking mouth in  order
to get the seal contact condition on the epidermis   part. In a version the spout is foreseen in elastic material to allow its fitting
in coaxial coupling through pressure on the other cylinder end.

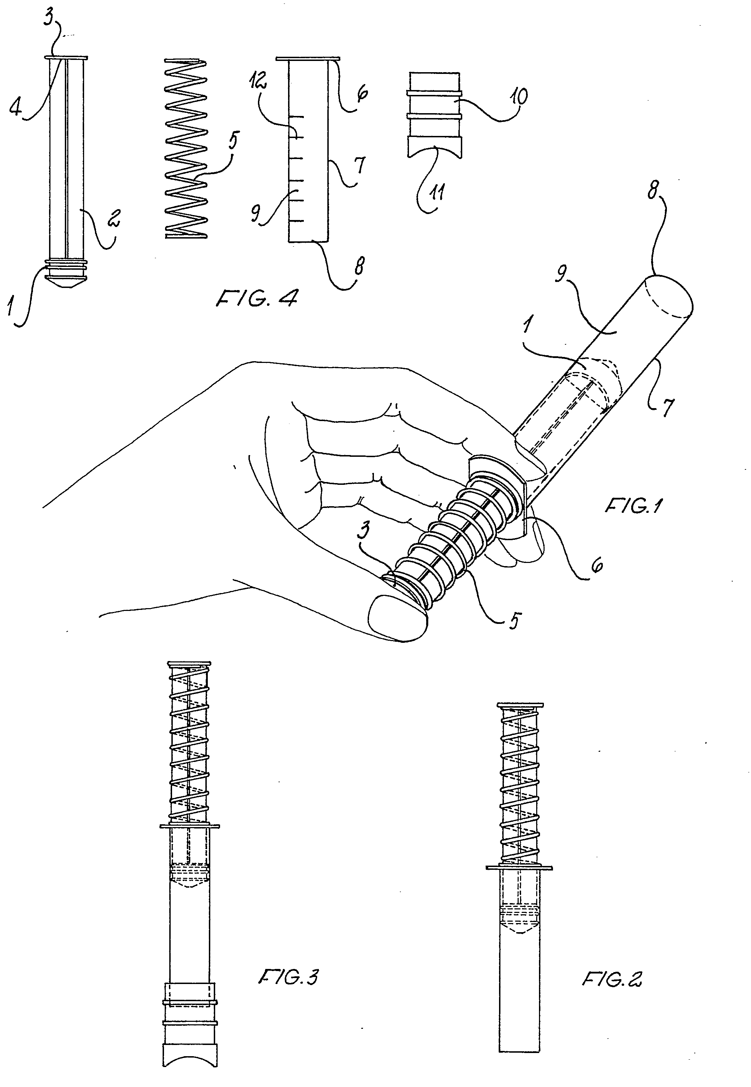

An execution form is illustrated, in a mere indicative way, by the dra
wings of table 1, where fig. 1 is the perspective view of the instrument in condition of hand taking in the restraint spring charging phase. The cylinder is restrained on the collar between the hand index
and middle finger, while the thumb brings the piston in introduction
by pressing its thrust base. Fig. 2 is the view of the instrument with
the dashed rod part and the piston introduced into the cylinder in star
ting position. In this condition, the helical spring operates with position restraint. Fig. 3 is the view of the same instrument with fitted
bent sucking opening. Fig. 4 is the separate view of the instrument com
ponents.

The seal piston 1 is employed which is held by rod 2 with thrust 3;
this one determines the underlying collar 4 for containing the helical spring 5 which, on the other end, is lying on collar 6 of cylinder 7 when the piston is axially imposed in it. For drawing out blood,

**0166010**

the spring 5 is loaded on the hit position by pressing the sucking o-pening 8 and the piston 1 is allowed to return by means of spring 5. This brings the blood to folow into the sucking chamber 9 as it comes out from the lanced point. In order to draw the blood from bent epidermis parts, beak 10 with bent sucking opening 11 has been foreseen. To establish the quantity of the blood drawn out, graduation 12 on the cylinder 7 has been foreseen in a version.

The particulars of execution, the form of the components, the materials and everything else on order may be foreseen in differente way.

- 1 -

0165010

Claims.

1) Device for the instantaneous sampling of the blood polluted by venomous reptiles bite, characterized by the fact that employs a cylindrical part in which a seal piston is introduced in initial position on the upper side, to be manually acted on teh thrust rod supporting it. The retaining condition is ensured by a helical spring fitted on the said rod between the collars of the thrust base and of the cylinder. When a limb has been injured, after having blocked the blood circulation in the part by binding it with a band and after having lanced the interested zone, the opera tor brings the retention spring under charge by introducing the pressed piston through the thumb action on the thrust base, while keeping the cylinder on its collar between two fingers. He then fits the cylinder sucking opening by pressure on the hit part and allows the piston to return in its position through its retention spring. The reduction of the air pressure in the sucking chamber of the cylinder causes the instantaneous coming out of some blood quantity according to the cylinder diameter and to the piston return course.

2) Device for the instantaneous sampling of the blood polluted by venomous reptile bite, according to the previous claim, characterized by the fact that this permits to determine the quantity of blood to draw out by foresee ing a relevant graduation for this purpose.

3) Device for the instantaneous sampling of the blood polluted by venomous reptile bite, according to the previous claims, characterized by the fact that in order to allow the blood drawing out from curved epidermic parts, such as they can be considered on the limbs, a cylindrical spout is fore seen fitted with a bent sucking mouth in order to get the seal contact condition on the epidermis part.

4) Device for the instantaneous sampling of the blood polluted by venomous reptile bite, according to the previous claims, characterized by the fact that in a version the spout is foreseen in elastic material to allow its fitting in coaxial coupling through pressure on the other cylinder end.

0166010

5) Device for the instantaneous sampling of the blood polluted by venomous reptile bite, according to the previous claims, characterized by the fact that the seal piston 1 is employed which is held by rod 2 with thrust base 3; this one determines the underlying collar 4 for containing the helical spring 5 which, on the other end, is lying on collar 6 of cylinder 7 when the piston is axially imposed in it.

6) Device for the instantaneous sampling of the blood polluted by venomous reptile bite, according to the previous claims, characterized by the fact that for drawing out blood, the spring 5 is loaded on the hit position by pressing the sucking opening 8 and then piston 1 is allowed to return by means of spring 5. This brings the blood to flow into the sucking chamber 9 as it comes out from the lanced point.

7) Device for the instantaneous sampling of the blood polluted by venomous reptile bite, according to the previous claims, characterized by the fact that in order to draw the blood from bent epidermis parts, beak 10 with bent sucking opening 11 has been foreseen.

8) Device for the instantaneous sampling of the blood polluted by venomous reptile bite, according to the previous claims, characterized by the fact that to establish the of the blood drwn out, graduation 12 on cylinder 7 has been foreseen in a version.

FIG. 4

FIG. 1

FIG. 3

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | GB-A-2 008 200 (EMERIT) <br> * Figures 1-4,8; page 1, line 103 - page 2, line 56; page 3, lines 3-28 * | 1,5,6 | A 61 B 5/14 |
| A | | 3,4 | |
| | --- | | |
| Y | FR-A-2 331 319 (VISCARO) <br> * Figures; page 1, lines 24-34 * | 1,5,6 | |
| | --- | | |
| A | GB-A-1 105 367 (MARBACH et al.) <br> * Figure 2; page 2, lines 40-44 * | 2,8 | |
| | --- | | |
| A | US-A-2 360 051 (EWESON) <br> * Figure 1; page 1, left-hand column, line 40 - right-hand column, line 55 * | 1,4 | |
| | ----- | | **TECHNICAL FIELDS SEARCHED (Int. Cl.4)** <br><br> A 61 B <br> A 61 M <br> A 61 D |

The present search report has been drawn up for all claims

| Place of search | Date of completion of the search | Examiner |
|---|---|---|
| THE HAGUE | 22-02-1985 | CHEN A.H. |